# EUROPEAN PATENT APPLICATION

(11) **EP 0 561 172 A1**
(43) Date of publication of application: **22.09.1993**
(21) Application number: 93102626.4
(22) Date of filing: 23.09.1987
(51) Int. Cl.: C12N 15/12, C12Q 1/68

(54) **Recombinant DNA Clone Encoding Laminin Receptor**

(30) Priority: 26.09.1986 US 911863
(62) Divisional of application: 87907048.0
(71) Applicant: THE UNITED STATES OF AMERICA as represented by the Secretary United States Department of Commerce, Springfield, Virginia 22161 (US); RHONE-POULENC RORER INTERNATIONAL (HOLDINGS) INC., Lewes, Delaware 19958 (US)
(72) Inventor: Sobel, Mark E., Bethesda, Maryland 20817 (US); Liotta, Lance A., Potomac, Maryland 20854 (US); Wewer, Ulla M., Rockville, Maryland 20852 (US); Jaye, Michael C., Arlington, Virginia 22204 (US); Drohan, William N., Springfield, Virginia 22152 (US)
(74) Representative: Lord, Hilton David

(57) **Abstract**

A recombinant cDNA clone encoding a cell matrix receptor specific for laminin which is expressed on the surface of carcinoma and epithelial cells is provided, from which hybridisation probes are useful in cancer diagnosis.

## Description

### BACKGROUND OF THE INVENTION

### Technical Field

The present invention is related generally to recombinant DNA. More particularly, the present invention is related to a recombinant cDNA clone encoding high affinity (about 10⁻⁸ to 10⁻¹⁰ Kd) cell surface receptors for laminin.

### State of the Art

Laminin is a major glycoprotein component of basement membranes and mediates the attachment of both epithelial and neoplastic cells to the basement membrane. The basement membrane is a ubiquitous, specialized type of extracellular matrix separating organ parenchymal cells from interstitial collagenous stroma. Interaction of cells with this matrix is an important aspect of both normal and neoplastic cellular processes. Normal cells appear to require an extracellular matrix for survival, proliferation, and differentiation, while migratory cells, both normal and neoplastic, must traverse the basement membrane in moving from one tissue to another. In particular, metastatic cancer cells arising is squamous or glandular epithelium must traverse the basement membrane to enter the circulatory and lymphatic systems (intravasation); the circulating neoplastic cells are typically arrested in the capillary beds of an organ, invade the blood vessel walls, and penetrate the basement membrane to extravascular tissue (extravasation), where a secondary neoplasm is then established. Laminin receptor, by mediating the attachment of both epithelial and neoplastic cells to the basement membrane, plays a critical role in controlling, inter alia, the metastatic process. U.S. Patent 4,565,789 describes the isolation and characterization of certain aspects of laminin receptor. But a cloned DNA sequence for ancoding cell surface receptor for laminin has not heretofore been known.

### SUMMARY OF THE INVENTION

It is, therefore, an object of the present invention to provide a recombinant cDNA clone capable of encoding high affinity (about 10⁻⁸ to 10⁻¹⁰ Kd) cell surface receptor for laminin.

It is an additional object of the present invention to provide synthetic fragments of laminin receptor which encode specific domains such as membrane-spanning and ligand binding regions.

It is another object of the present invention to provide a diagnostic method to assess the content of laminin receptor mRNA in different tumor cell and normal tissue cell populations.

It is a still further object of the present invention to provide a diagnostic method to determine the pattern of laminin receptor genes in different tissue and tumor cell populations.

### BRIEF DESCRIPTION OF DRAWINGS

These and other objects, features and many of the attendant advantages of the invention will be better understood upon a reading of the following detailed description when considered in connection with the accompanying drawings wherein:

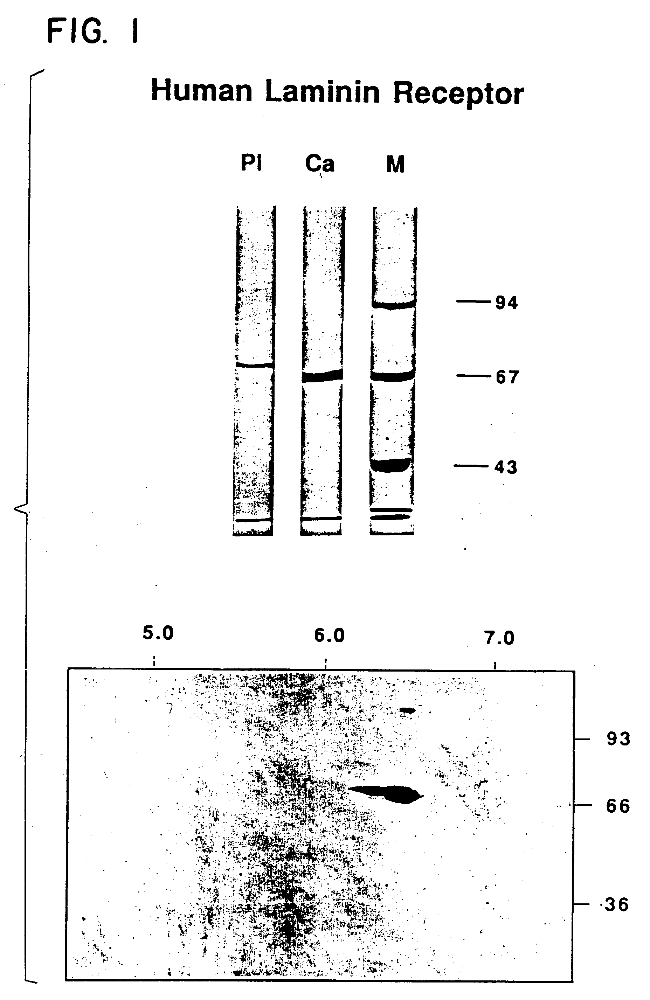

FIG. 1 demonstrates the homogeneity of purified laminin receptor. TOP shows comparison of the purified laminin receptor from colon carcinoma and from normal human placental tissue. Materials eluted with 1 M NaCl from the laminin-Sepharose affinity column were analyzed by NaDodSO₄-polyacrylamide gel electrophoresis (7%) in the presence of dithiothreitol and visualized with silver staining. The panel exemplifies the slight variability in the molecular weight (68,000-72,000) between preparations of the laminin receptor. Lane P1: Laminin receptor (1 µg) isolated from human placental tissue. Lane Ca: Laminin receptor (3 µg) isolated from human colon carcinoma. Lane M: Molecular weight markers: phosphorylase b (94,000), bovine serum albumin (67,000), and ovalbumin (43,000).
   BOTTOM shows isoelectric focusing and two-dimensional gel electrophoresis of the purified laminin receptor. The colon carcinoma laminin receptor protein sample (0.1 µg) shown above was radiolabeled with I¹²⁵ and subjected to isoelectric focusing (pH range 5-7) and two-dimensional gel electrophoresis on a 10% NaDodSO₄ -polyacrylamide gel. Molecular weight markers: phosphorylase b (93,000), bovine serum albumin (66,000), glyceraldehyde 3-phosphate dehydrogenase (36,000);

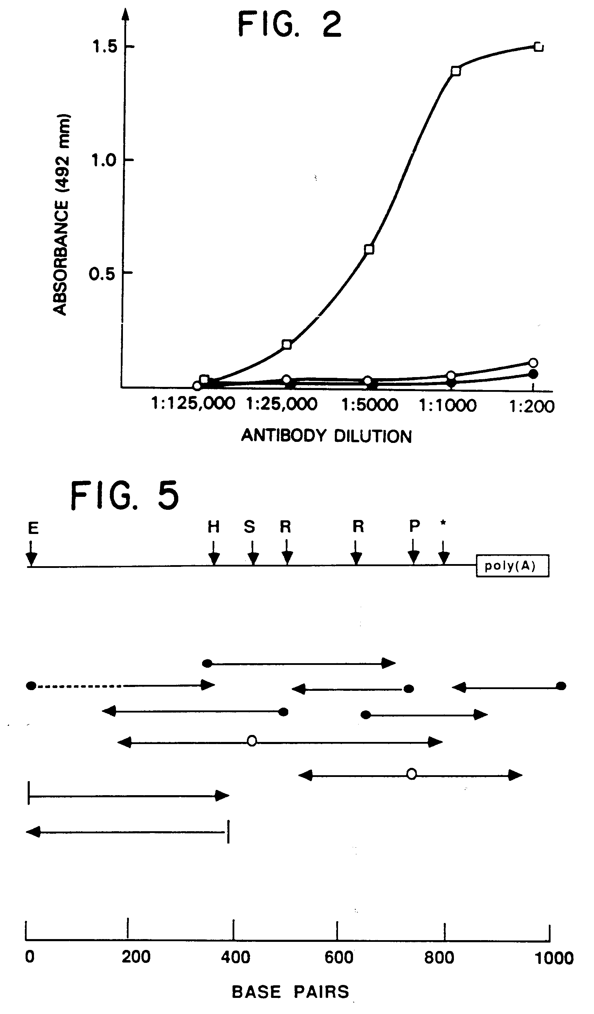

FIG. 2 shows an ELISA of the purified laminin receptor. Each microtiter well was coated with purified tumor laminin receptor and reacted for 2 hours against LR1 monoclonal antibody 2H5 (□), anti-albumin (O) or anti-α-amylase moroclonal antibody (●). Antibodies were detected using peroxidase-conjugated goat anti-mouse IgM or peroxidase-conjugated swine anti-rabbit IgG;

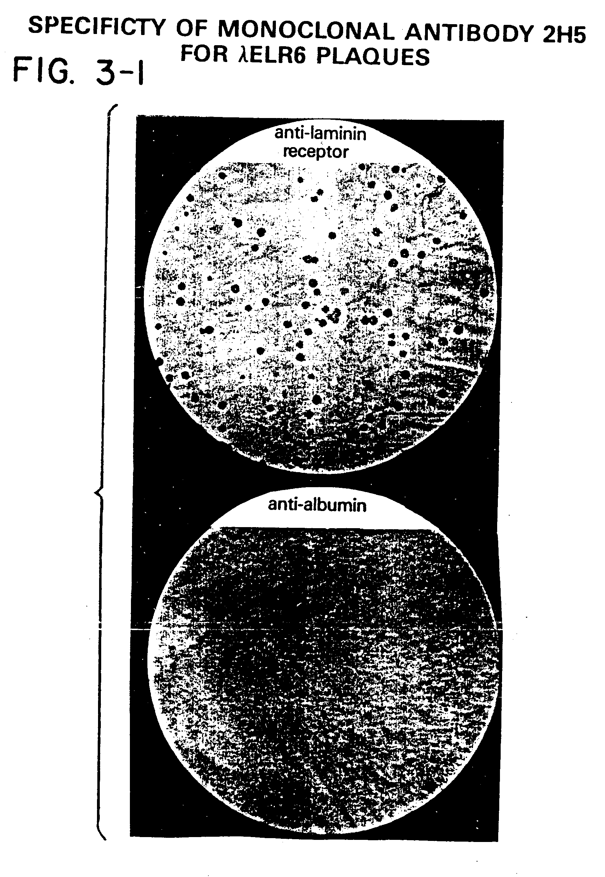

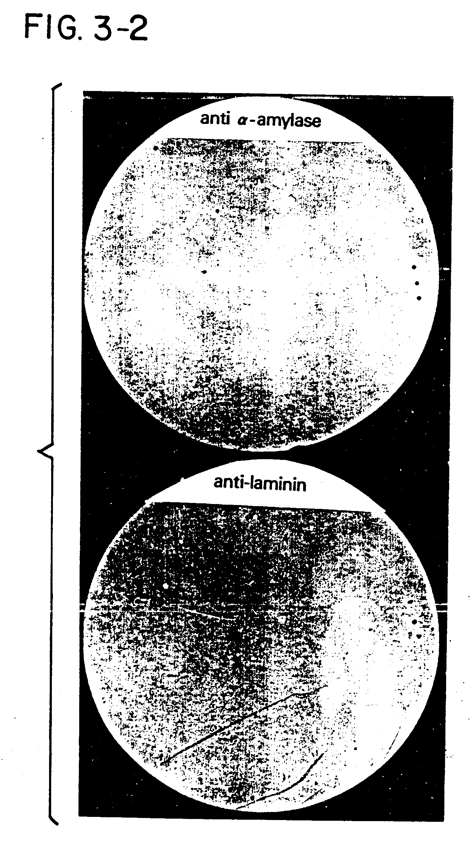

FIG. 3 depicts the specificity of the anti-laminin receptor monoclonal antibody for λELR6 plaques. Filters containing approximately 50-100 purified λELR6 plaques were reacted with either anti-laminin receptor monoclonal antibody, anti-α-amylase monoclonal antibody, anti-albumin antibody or anti-laminin antibody. Antibody binding was detected using peroxidase-conjugated second antibody;

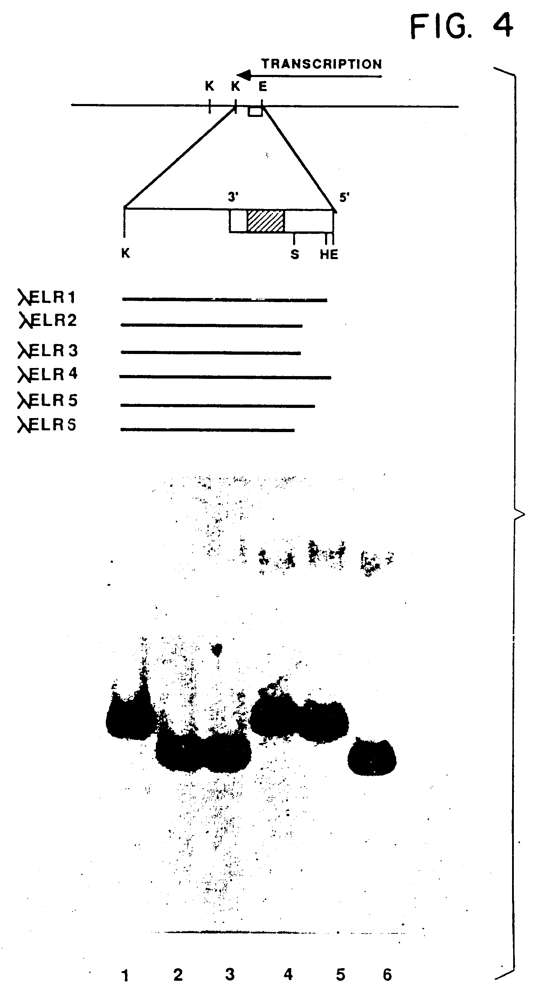

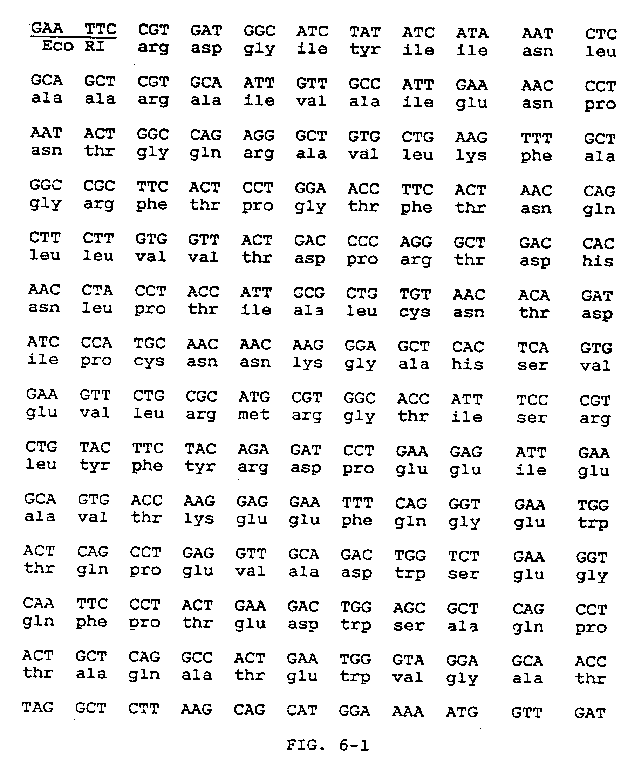

FIG. 4 demonstrates the overlapping laminin receptor cDNA inserts of λELR1-6. The top line is a diagram of prototype λELR recombinant λgt11 phage, showing the two KpnI sites (K) to the left of the cDNA inserts. The box depicts the cDNA insert. The left EcoRI site in each insert was not present. The right EcoRI site (E) is shown. Transcription from the lacZ gene of λgt11 proceeds from the right arm of the phage toward the left, through E. The region extending from the KpnI site (closest to the cDNA insert) to the EcoRI site of the largest cDNA insert (λELR4) is expanded and shown below. The stippled area in the cDNA insert box designates the PstI-SphI restriction fragment isolated from a subclone of λELR4 (see FIG. 5) which was nick translated and used as a hybridization probe. The common SacI (S) and HindIII (H) sites described in the text are indicated. Phage DNA from each of the ELR recombinant phage was restricted with KpnI and EcoRI, electrophoresed through a 1% agarose gel, transferred to nitrocellulose, and stringently hybridized to the PstI-SphI restriction fragment described above. The length of the KpnI-EcoRI restriction fragment from each of the λELR recombinant phage which hybridized to the probe is diagrammed above the radioautograph of the blot, and was determined by comparison to the standard λ/HindIII digests;
FIG. 5 diagrams the strategy to sequence the cDNA insert of λELR4. The restriction map of subcloned insert of λELR4 is shown on top. E is the 5' EcoRI site of the insert. H: HinfI, S: SphI, R: RsaI, P: PstI, *: ochre termination codon. The arrows indicate the sequencing direction of fragments labeled either by kinasing at the 5' end with (γ-P³²) ATP (●) or by tailing at the 3' end with (α-P³²) dideoxy ATP (O) or by the dideoxy synthesis method ( 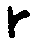 ). Subclones used for sequencing were pLR4-1, pLR4-2, and pLR4-4;

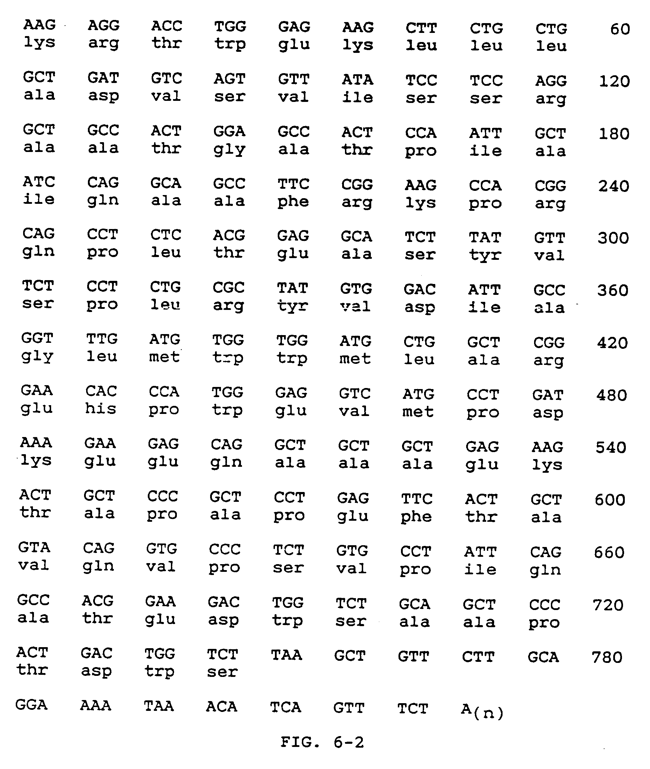

FIG. 6 provides the nucleotide sequence of the λELR cDNA insert with derived protein sequence below. Shown to the right of each line is the number of base pairs from the artificial EcoRI site (bases 1-6);

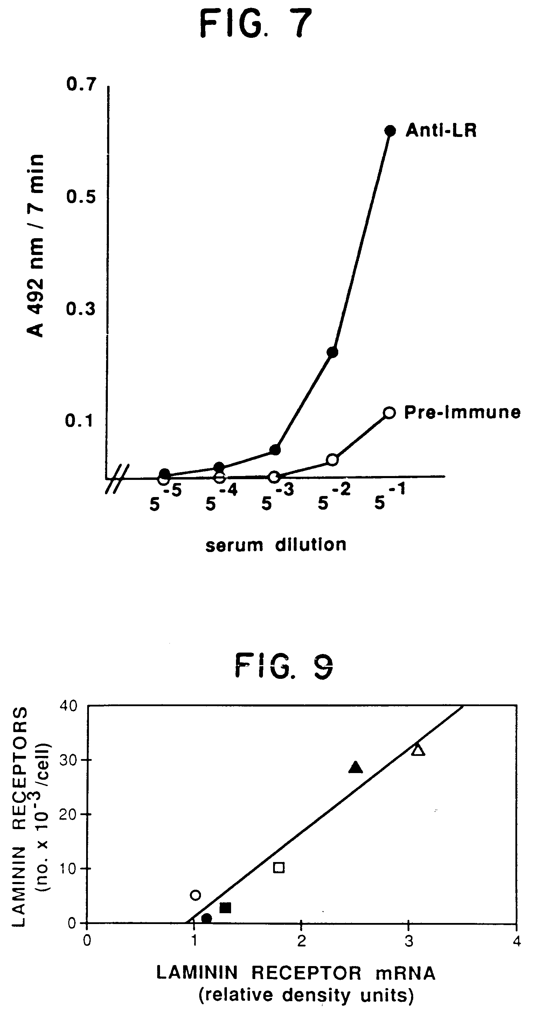

FIG. 7 shows ELISA determination of synthetic laminin receptor peptide. Synthetic laminin receptor peptide RTLR2 was generated from the cDNA sequence in FIG. 6 and was assayed by ELISA using different dilutions of rabbit anti-laminin receptor antiserum or perimmune serum;

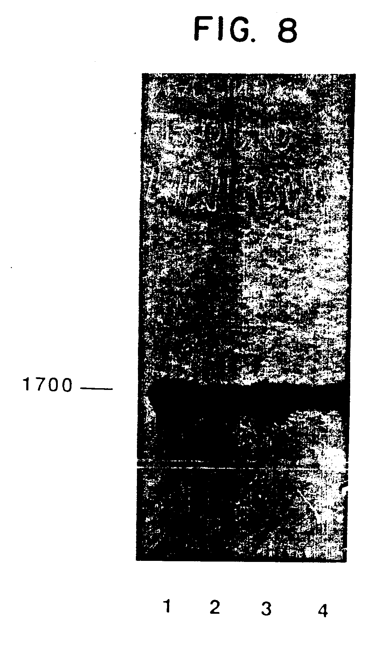

FIG. 8 shows an RNA gel blot hybridization. Total cellular RNA was extracted from several breast carcinoma cell lines, separated on methylmercury agarose gels and transferred to diazobenzyloxymethylcellulose paper. The filter was hybridized to nick translated ( ³²P )-labeled EcoRI-PstI insert from pLR4-1. The length of the hybridized mRNA species was determined by comparison with the known sizes of rRNA and with the sizes of λDNA restricted with HindIII. Lane 1: MCF-7 (parent); Lane 2: MCF7-3E5; Lane 3: MCF7-5H7; Lane 4: ZR75;

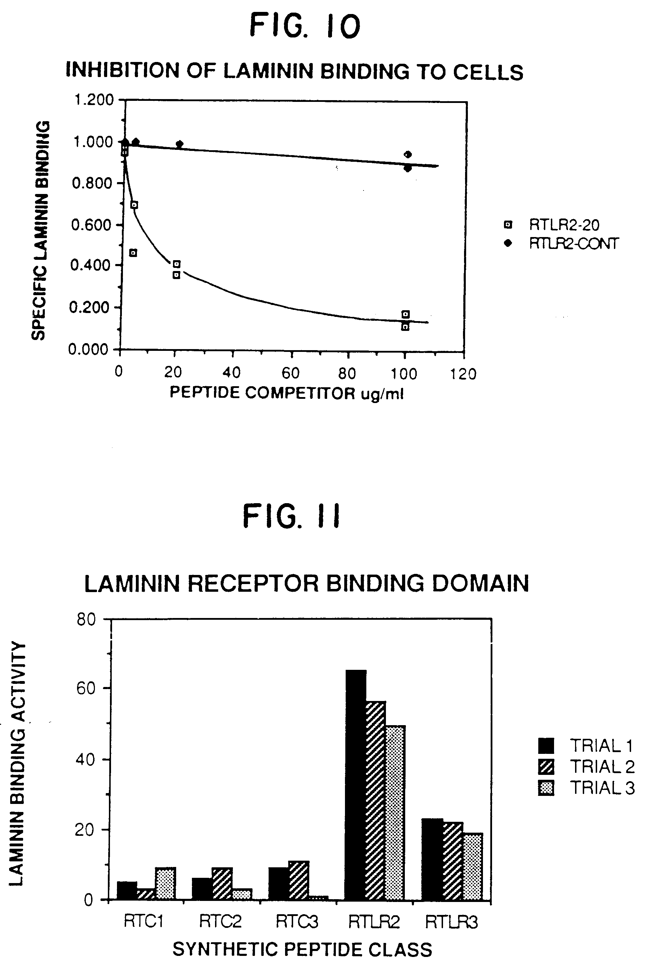

FIG. 9 shows the correlation of laminin receptor mRNA levels with ability of six human carcinoma cell lines to bind laminin. Different timed exposures of radioautographs such as shown in FIG. 8 were measured densitometrically to determine a linear response range. The lowest amount of RNA hybridized in the series was assigned the number 1.0 and all other values were calculated relative to that value. The number of laminin receptors per cell for each cell line was calculated from Scatchard plots of specifically bound (I¹²⁵) laminin to log growth phase cells. A linear regression analysis revealed a correlation coefficient of 0.97. MCF-7 parent □, MCF-7 Clone 5H7 △, MCF-7 Clone 3E5 ■ , ZR-75 O, renal carcinoma A-704 ●, Panc-1 ▲ ;
FIG. 10 demonstrates that synthetic laminin receptor peptide inhibits laminin binding to cells. The ability of A2058 human melanoma cells to bind specifically to laminin was tested in the presence of various amount of RTLR2 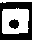 or RTLR2-control ♢ fragments generated from the cDNA sequence of FIG. 6. Values are expressed relative to the amount of laminin bound in the absence of added fragment; and
FIG. 11 shows identification of synthetic peptide laminin receptor fragments involved in ligand binding. Peptide fragments generated from cDNA sequence with predicted reverse turn conformation (RTLR2, RTLR3) as well as control peptides were bound to glass beads and incubated with (I¹²⁵) laminin. The values shown are the number of cpm x 10⁻³ bound to beads after subtraction of cpm bound to beads without fragment.

### DETAILED DESCRIPTION OF THE INVENTION

The above and various other objects and advantages of the present invention are achieved in accordance with Claims 1 to 8.

Unless defined otherwise, all technical or scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned hereunder are incorporated herein by reference.

The methodology for constructing and isolating the recombinant cDNA clone of Figure 6 is described in further detail in Example II and includes the following general steps, involving standard techniques well known in the art:
a) cDNA is synthesized using mRNA template from human umbilical vein endothelial cells.
b) Endothelial cell cDNA is inserted into the λgt11 vector (ATCC 37194).
c) The recombinant λgt11 is packaged and used to infect Escherichia coli 1090 cells (ATCC 37197).
d) The resulting λgt11 human endothelial cell cDNA expression library is screened with a monoclonal antibody which recognizes a domain of the human laminin receptor involved in binding of laminin.
e) Six plaques, designated λELR1-6, are thus isolated and purified (FIG. 3). The cDNA inserts are analyzed by restriction endonuclease mapping and are found to have a common domain, indicating that they encode the epitope of the laminin receptor recognized by the monoclonal antibody (FIG. 4).
f) The largest cDNA insert of the six purified phase is subcloned into plasmid vectors to facilitate further analysis and DNA sequencing. The EcoRI site linking the cDNA insert to the left arm of λgt11 is missing in all six clones. A PvuII site 11 bp downstream is therefore utilized to purify cDNA insert from the largest recombinant phage, λELR4. The EcoRI-PvuII restriction fragment of λELR4 is subcloned into the EcoRI-PvuII sites of pBR322 to make pLR4-1.

A deposit of the recombinant cDNA clone pLR4-1 transformed into host E. coli strain C600r⁻m⁻ (ATCC 33525) obtained in accordance with the present invention has been made at the American Type Culture Collection (ATCC), Rockville, MD, under accession number 67199. Upon request, the Commissioner of the PTO shall have access to the deposit, and upon issuance of the patent, this deposit will continue to be viably maintained for at least 5 years after the last request or at least 30 years or the life of the patent and made available to the public without restriction, of course, consistent with the provisions of the law.

The method for definitively identifying the present invention was based on comparison of cDNA sequence of the recombinant plasmids (FIG. 6) with the amino acid sequence of a cyanogen bromide-generated octapeptide of purified placental laminin receptor. The methodology for obtaining octapeptide amino acid sequence is described in further detail in Example I and includes the following general steps, involving standard techniques well known in the art:
a) Microsomal membranes from human placental tissue are prepared and solubilized.
b) The solubilized microsomal membrane-containing preparation is passed through an affinity matrix of purified laminin-Sepharose 4B and after extensive washing, the high affinity laminin receptor protein is eluted from the ligand by high salt (1 M NaCl).
c) The homogeneity of the human placental laminin receptor is assayed by two dimensional gel electrophoresis (FIG. 1) and enzyme-linked immunosorbent assay (ELISA) (FIG. 2).
d) The human placental laminin receptor is cleaved with cyanogen bromide and the resulting peptides are fractionated by reverse phase high pressure liquid chromatography (HPLC).
e) Microsequence analysis of a placental oligopeptide reveals an octapeptide with the unique sequence Met-Leu-Ala-Arg-Glu-Val-Leu-Arg.

An alternate method for identifying the present invention was based on the ability of rabbit antiserum directed again human metastatic breast carcinoma laminin receptor to identify (by ELISA), a synthetic peptide generated from the cDNA sequence of pLR4-1 (FIG. 7). The methodology for obtaining the anti-laminin receptor antiserum is described in further detail in Example IV and includes the following general steps, involving standard techniques well known in the art:
a) Laminin receptor is purified from human metastatic breast carcinoma as described supra for placental laminin receptor.
b) Homogeneity of the tumor laminin receptor is assessed as described supra for placental laminin receptor.
c) Rabbits are immunized by subcutaneous injections of tumor laminin receptor cut out of NaDodSO₄-polyacrylamide gels.

Diagnostic assays for cells containing laminin receptor mRNA, such as human cancer cells, have been developed as useful tools in the diagnosis and prognosis of cancer. The method for assaying laminin receptor mRNA content is described in further detail in Example V and includes the following general steps, following standard techniques well known in the art:
a) Total cellular RNA is isolated from tissue specimen or cells.
b) RNA is denatured and electrophoresed through denaturing agarose gels and transferred to a filter paper.
c) The filter paper containing the RNA is hybridized to radiolabeled laminin receptor cDNA.
d) After washing, the filter is exposed to X-ray film for sufficient time to obtain an image of the radioactive probe.
e) The relative intensity of the band on the film is a measure of the content of laminin receptor mRNA (FIG. 8).
f) Alternatively, in lieu of step (b) above, a small aliquot of denatured RNA can be directly bound to filter paper followed by steps (c) thru (e).
g) Radiolabeled laminin receptor cDNA can also be hybridized in situ to tissue sections and cells to determine specific cell populations expressing laminin receptor mRNA.

Increased content of laminin receptor mRNA in metastatic cells lends to the development of diagnostic assays for cells containing altered laminin receptor DNA. The methodology is based on the possible presence of restriction fragment length polymorphisms as has been found for a variety of genetic diseases. Amplification of laminin receptor genes in the genome of tumor or metastatic cells may also be assessed. The method for analyzing laminin receptor genomic DNA includes the following general steps, following standard techniques well known in the art:
a) High molecular weight genomic DNA is isolated from tissue specimen or cells, as described in Example VI.
b) DNA is digested with a variety of restriction endonucleases, electrophoresed through agarose gel and transferred to filter paper.
c) The filter containing the DNA is hybridized to radiolabeled laminin receptor cDNA.
d) After washing, the filter is exposed to X-ray film and the pattern of bands on the film is compared to that from normal tissue.

The methods described herein are based on the observation that the basement membrane is a continuous structure in benign neoplasms, and that laminin receptors on benign cells are occupied by attachment to basement membrane. In contrast, the basement membrane adjacent to invading tumor cells is not continuous. Invading tumor cells thus may have increased numbers of laminin receptors expressed on the cell surface which are not bound to ligand. It is the availability of laminin receptor sites on metastasizing carcinoma cells which is an important concept in the management and diagnosis of cancer. The aggressiveness of a carcinoma, defined as the tendency for a tumor cell of that carcinoma to migrate out of the primary tumor site into adjacent normal tissue and its propensity for spreading to distant sites in the body to initiate metastatic clones, is, then, in part, a reflection of the number of invasive cells with more available laminin receptors.

Based on the above, it is clear that the present invention can be used in a variety of ways in the diagnosis of cancer, as well as other diseases resulting from abnormal recognition of laminin by cells.

Specific examples to illustrate the present invention are now described.

### EXAMPLE I

### Isolation of Human Laminin Receptor cDNA Clones

### A. Materials and Methods

1. Cell Culture - Monolayer cultures of human umbilical vein endothelial cells were grown as described by Jaye, et al., Science, 228:882-885 (1985).
2. Preparation of RNA - Total cellular RNA was extracted from cell layers in culture by the guanidinium isothiocyanate-cesium chloride density method described by Chirgwin, et al., Biochemistry, 18:5294-5299 (1979). Poly (A)-containing RNA was isolated by chromatography on oligo(dT) cellulose (Type 3, Collaborative Research) according to the method of Aviv and Leder, Proc. Natl. Acad. Sci., 69:1408-1412 (1972).
3. Synthesis of cDNA - Oligo(dT)-selected human endothelial RNA was used as template for the synthesis of cDNA by the nethod of Buell, et al., J. Biol. Chem., 253:2471-2482 (1978) and double stranded cDNA was prepared as described by Wickens, et al., J. Biol. Chem., 253:2483-2495 (1978). The cDNA was made blunt ended with S1 nuclease as described by Ullrich, et al., Science, 196:1313-1319 (1977) and subsequent treatment with the Klenow fragment of E. coli DNA polymerase I as described by Wartell and Resnikoff, Gene, 9:309-319 (1980). The cDNA was then modified with EcoRI methylase, ligated to EcoRI linkers and exhaustively digested with EcoRI as described by Huynh, et al., DNA Cloning Volume 1: a practical approach, ed., D.M. Glover, IRL Press Ltd., Oxford, England, pp. 49-78 (1985).
4. Construction and Screening of Human Endothelial λgt11 cDNA Library - EcoRI-linked cDNA was ligated to EcoRI-digested and phosphatase-treated λgt11 as described by Huynh (1985) supra. The recombinant phase were packaged as described by Enquist and Sternberg, Methods Enzymol., 68:281-298 (1979) and amplified in E. coli Y1088 (ATCC 37195) as described by Huynh (1985) supra. E. coli Y1090 (ATCC 3/19) cells were infected with the endothelial cell λgt11 cDNA library ano 1.5 million plaques were screened by antibody recognition of a laminin receptor β-galactosidase fusion protein, as described by Young and Davis, Science, 222:778-782 (1983), using anti-laminin receptor monoclonal antibody 2H5 (Liotta, et al., Exp. Cell Res., 156:117-126 (1985). This antibody recognizes or interferes with the ligand binding of the laminin receptor (Liotta, et al. (1985) supra and Togo, et al., Basement Membranes, ed. Shibata, S., Elsevier, New York, pp. 325-333 (1985)). Antibody binding was detected using peroxidase-conjugated affinity-purified rabbit anti-mouse IgM as described supra in Example 1A4. Positive plaques were identified, amplified, and rescreened to purity as described by Benton and Davis, Science, 196:180-182. Filters containing plaques were reacted against control antibodies, including anti-human α-amylase and anti-human albumin (as described in Example 1A4 supra) as well as anti-rat laminin (Albrechtsen, et al., Cancer Res., 41:5076-5081 (1981)).
5. Southern Blot Hybridization - Phage DNA was isolated as described by Maniatis, et al., Molecular Cloning: A Laboratory Manual, Cold Spring Harbor Laboratory, Cold Spring Harbor, New York, pp. 77-85 (1982). Restriction endonuclease-digested DNAs were electrophoresed through agarose gels, transferred to nitrocellulose paper, and hybridized by the method of Southern, Methods Enzymol., 68:152-176 (1979). ³²P-labeled probes were prepared by nick translation as described by Maniatis, et al., Proc. Natl. Acad. Sci., 72:1184-1188 (1975).
6. Subcloning and Preparation of Plasmids and cDNA Insert - The EcoRI site linking the cDNA insert to the left arm of λgt11 was missing in all six clones. A PvuII site 11 bp downstream was therefore utilized to purify cDNA insert from the largest recombinant phage ELR4. The EcoRI-PvuII restriction fragment of λELR4 was subcloned into the EcoRI-PvuII sites of pBR322 to make pLR4-1 and into the EcoRI-HindII polylinker sites of pUC8 to make pLR4-2. Subsequently, to facilitate cDNA sequencing away from the long poly(A) tail, the EcoRI-PstI restriction fragment of pLR4-1 was subcloned into the EcoRI-PstI sites of pBR322 to make pLR4-4. Recombinants of pBR322 were transformed into E. coli C600r m (ATCC 33525) and pUC recombinants were transformed into E. coli JM 103 (Messing, et al., Nucleic Acids Res., 9:309-321 by the calcium chloride procedure of Mandel and Higa, J. Mol. Biol., 53:159-162 (1970)). Supercoiled plasmid DNA was recovered by alkali lysis after equilibrium centrifugation in ethidium bromidecesium chloride according to the procedures of Birnboin and Doly, Nuclei Acids Res., 7:1513-1523 (1979). cDNA inserts were isolated from restricted DNA and elutad from acrylamide gels as described by Sobel, et al., Proc. Natl. Sci., 75:5846-5850 (1978).

### B. Results

1. Selection of Laminin Receptor cDNA by Antibody Recognition - The human endothelial cell λgt11 cDNA library was screened using anti-laminin receptor monoclonal antibody 2H5. As described in Materials and Methods, supra, this antibody (a) specifically recognizes the laminin receptor on immunoblots, (b) recognizes the purified laminin receptor by ELISA (FIG. 2), (c) blocks the binding of laminin to either plasma membranes or cells, and (d) blocks attachment of cells to amnion basement membrane. These properties clearly indicate that the monoclonal antibody recognizes or interferes with the laminin binding domain of the laminin receptor. Six plaques were initially selected. After plaque purification, all six phage designated λELR1-6, showed an intense reaction with the anti-laminin receptor monoclonal antibody, but showed no reactivity toward a class matched (IgM) monoclonal antibody directed against human α-amylase, nor toward antibodies directed against laminin or against proteins similar in size to laminin receptor such as albumin (FIG. 3).
2. Common Domain of λELR1-6 - Restriction endonuclease digestion of the six phase, λELR1-6, revealed that the EcoRI site linking the 3' end of the cDNA insert to the left arm of λgt11 was missing in all six phage. Single, double, and triple endonuclease digestions using SacI, KpI, HindIII, and EcoRI were performed to map λELR1-6. These experiments, summarized in FIG. 4, indicated that (a) the sizes of the cDNA inserts ranged from about 450 to 975 bp, (b) all the clones shared an internal SacI site approximately 450-500 bp from the 3' end of the clones, (c) the insert of the λELR6 extended only about 20 bp 5' to the internal SacI site, and (d) the inserts of λELR1 and λELR4 shared a HindIII site approximately 30 and 40 bp, respectively, from the 5' EcoRI site. The six recombinant phage thus have a common 450-500 bp region, extending from just 5' to the SacI site to the 3' end of each cDNA insert, suggesting that this region encodes the domain recognized by the monoclonal antibody 2H5. This observation was tested by Southern blot experiments in which KpnI-EcoRI and KpnI-SacI double digests of the DNA from each of the six recombinant phage were stringently hybridized to a PstI-SphI restriction fragment isolated from λELR4. A representative hybridization experiment, shown in FIG. 4, shows that a common 450-500 bp region of all six recombinant phage encodes the antigenic domain recognized by monoclonal antibody 2H5.

### EXAMPLE II

### cDNA Sequence Determination

### A. Materials and Methods

1. DNA Sequencing - The cDNA from λELR4 was sequenced according to the strategy shown in FIG. 5. Both the chemical modification method of Gilbert and Maxam, Proc. Natl. Acad Sci., 70:3581-3584 (1973) were used. In the former case, cDNA restriction fragments of subclones pLR4-1, pLR4-2, and pLR4-4 described in Example IIA6, supra, were labeled either by treating the 5' end with T4 polynucleotide kinase (Boehringer-Mannheim) and (γ-P³²)ATP as described by Gilbert and Maxam (1973), supra, or by tailing the 3' end with (α-P³²) dideoxy ATP using a 3' tailing kit (Amersham). In the case of the dideoxy synthesis method, the EcoRI-SacI restriction fragment of ELR4 was subcloned into M13mp18 and M13mp19 (Yanisch-Perran, et al., Gene, 33:103-119 (1981)).

### B. Results

1. Nucleotide and Deduced Amino Acid Sequence of the cDNA insert of λELR4 - Sequence of the entire cDNA insert of λELR4 reveals a 253 amino acid open reading frame consistent with the right arm EcoRI insertion site of the λgt11 vector (FIG. 6). The sequence of the "common domain" recognized by the 2H5 epitope (see Example IIB2, supra) extends from nucleotide 361 to nucleotide 765 in FIG. 6. It includes an octapeptide-encoding sequence (nucleotides 409-432) identical to that of the purified cyanogen bromide-generated oligopeptide of placental laminin receptor described in Example Ib2, supra. Notable within this sequence is a six amino acid repeat separated by 3 amino acids toward the carboxy terminus of the laminin receptor. The cDNA sequences which encode the amino acid repeats are not identical (nucleotides 670-687 and 697-714). Following an ochre stop codon, the λELR4 cDNA insert includes a 3' untranslated region of 66 bp, including a canonical polyadenylation signal AATAAA 17 bp upstream from a long poly(A) stretch. After subtracting the length of the 3' untranslated region and the poly(A) tail, the largest possible extent of the 2H5 epitope, as defined by the common cDNA region of λELR1-6, can be narrowed down to 393 bp or 131 amino acids. Computer analysis by the method of Wilbur and Lipman, Proc. Natl. Acad. Sci, 80: 726-730 (1983) reveals no significant homologies to known protein sequences.

It is noted that a potential membrane-spanning domain of the laminin receptor could include an eleven amino acid hydrophobic polypeptide encoded by nucleotides 52-84 of FIG. 6.

### EXAMPLE III

### Hybridization of Laminin Receptor cDNA to RNA

### A. Materials and Methods

1. Cell Lines - The MCF-7 human breast cancer cell line (parent) was provided by the Michigan Cancer Foundation. Two cloned MCF-7 cell lines MCF7-5H& and MCF7-3E5, were obtained from P. Horan Hand (National Cancer Institute) and are described by Greiner, et al., Int. J. Cancer, 36:159-166 (1985). The ZR-75 human breast carcinoma line is described by Engel and Young, Cancer Res., 38:4327-4339 (1978) and was obtained from L. Engel (National Cancer Institute) and the human renal carcinoma line A-704 was from S. Aaronson (National Cancer Institute) and is described by Giard, et al., J. Natl. Cancer Inst., 51:1417-1423 (1973). The human Panc-1 cell line was obtained from the American Type Culture Collection. Murine NIH-3T3 cells and its Kirsten virus-transformed derivative (KNIH) were obtained from M. Gottesman (National Cancer Institute) and their growth is described by Gottesman, Proc. Natl. Acad. Sci., 75:27657-2771 (1978). Murine F9 tertocarcinoma cells and their dibutyrylcyclic AMP plus retinoic acid differentiated derivatives (Strickland, et al., Cell, 21:347-355 (1980)) were from W. Anderson (National Cancer Institute). The rat L2 cell line is described by Wewer, Dev. Biol., 93: 416-421 (1982).
2. Preparation of RNA - RNA was prepared from cell cultures as described in Example IA2, supra, or by the guanidine-hydrochloride method described by Strohman, et al., Cell, 10:265-273 (1977).
3. Northern Hybridization - 5 µg of total cellular RNA were separated on methylmercury/agarose gels and transferred to diazobenzyloxymethylcellulose paper (Schleicher and Schuell) by the method of Alwine, et al., Proc. Natl. Acad. Sci., 74:5350-5354 (1977) as described by Sobel, et al., Biochemistry, 20:2678-2684 (1981). Filters were hybridized to nick translated cDNA insert as described in Example IA5, supra. Various time exposures of radioautographs were measured densitometrically to determine linear-response range. The blots were conter-screened with actin cDNA probe (described by Cleveland, et al., Cell, 20:95-105 (1980)) and with heat shock cDNA probe (described by Hickey, et al. Gene, 43:147-154 (1986)) to ensure equal amounts of RNA were transferred.
4. Laminin-Binding Assays - The number of laminin receptors per cell for each cell line was calculated from Scatchard plots of specifically bound (¹²⁵I)-labeled laminin to logarithmic growth phase cells as described by Liotta, et al., Exp. Cell Res., 156:117-126 (1985).

### B. Results

1. Expression of Human Laminin Receptor mRNA -It was previously reported that the amount and surface distribution of laminin receptor is different in various carcinomatous human tissues (Horan Hand, et al., Cancer Res., 45:2713-2719 (1985)). In general, malignant tissues have more unoccupied laminin receptors on their cell surface and bind to more laminin than do their more benign counterparts. To determine if laminin receptor mRNA levels play a role in determining the amount of cell surface laminin receptors available for ligand binding, a Northern blot experiment was performed (FIG. 8). The laminin receptor cDNA insert recognizes a mRNA of about 1700 bases which is sufficient in length to encode a protein with the estimated size of laminin receptor. The level of hybridized RNA from a variety of human epithelial cell lines varied. In particular, there was greater hybridization to RNA from the metastatic breast carcinoma cell line MCF-7 than to RNA from the nonmetastatic breast carcinoma cell line ZR-75. In general, the level of hybridized RNA correlated directly with laminin binding assays (Fig. 9), suggesting that the amount of laminin receptor mRNA available for the biosynthesis of receptor may be a rate-limiting control step in the regulation of cellular attachment to laminin the basement membrane. Hence, the determination of laminin receptor mRNA levels in human tumor tissue can be used diagnostically to predict the relative aggressiveness of the tumor or suspectibility to therapeutic regimens based on laminin receptor content. As mentioned above, this can be accomplished by Northern blot hybridization or in situ hybridization of sectioned tumor material, using laminin receptor cDNA as probe. Laminin receptor cDNA may be prepared as probe for the above mentioned procedures by a variety of means following standard techniques well known in the art. The nick translation procedure described in Example IIA5 can be used to radiolabel cDNA insert with either ( ³²P), (³⁵S), or (³H). Alternatively, the laminin receptor cDNA insert can be subcloned into plasmids permitting transcription of radiolabeled RNA by methods described by Melton, et al., Nucleic Acids Res., 12:7035-7056 (1984). Biotinylated RNA and DNA hybridization probes in conjunction with chromogenic detection systems have also been described, for example, by Langer, et al., Proc. Natl. Acad. Sci., 78:6633-6637 (1981) and by Leary, et al., Proc. Natl. Acad. Sci., 80:4045-4049 (1983).
2. Detection of Laminin Receptor mRNA in Other Species - Northern blots containing RNA from rat L2 cells and from murine cells such as NIH 3T3 and F9 teratocarcinoma and their derivatives also detected a mRNA of about 1700 bases. Thus, the human laminin receptor cDNA insert can cross hybridize to RNA from other vertebrate species (data not shown).

### EXAMPLE IV

### Polymorphisms of Laminin Receptor

### A. Materials and Methods

1. Identification of Cross-Hybridizing Laminin Receptor cDNAs - The human endothelial cell λgt11 cDNA library described in Example IA4 was used to infect E. coli Y1088 cells (ATCC 37195) and screened using ³²P-labeled cDNA insert from λELR4 by the plaque hybridization procedure of Benton and Davis, Science, 196:180-182 (1977). Positive plaques were identified amplified, and rescreened to purity as described by Benton and Davis, supra.
2. DNA Sequence Determination - The cDNA inserts of positive phage here subcloned into the EcoRI site of pUC vectors as described in Example IA6, and cDNA sequence was determined by the method of Gilbert and Maxam, Proc. Natl. Acad. Sci., 70:3581-3584 (1973).
3. Isolation of Human Genomic DNA - Genomic DNA was isolated from human cell lines described in Example IIIA1 supra by a gentle digestion with sodium dodecylsulfate and proteinase K as described by Blin, et al., Nucleic Acids Res., 3:2303-2308 (1976).
4. Southern Hybridizations - Genomic DNA from the cell lines described above as well as from human liver (obtained from M. Young, National Institute of Dental Research) was digest exhaustively with EcoRI, HindIII, BamHI, or XbaI, electrophoresed on agarose gels, transferred to nitrocellulose and hybridized to λELR4 cDNA insert as described in Example IIA5, supra.

### B. Results

1. cDNA Sequence Comparison - Partial cDNA sequences were determined for recombinant phage λELR10, λELR14, λELR106, λELR112. The sequence from the 5' EcoRi site to the internal SacI site of λELR14 completely corresponds to nucleotides 7-381 of FIG. 6 and includes 96 additional bases upstream from λELR4. The other clones analyzed show high homology to λELR4 sequences but contain multiple point mutations. For each of the other clones, there are no open translational reading frames, suggesting that they may be cDNAs of pseudolaminin receptor genes.
2. Genomic DNA Analysis - Southern blot analysis of high molecular weight genomic DNA from human liver and from a variety of human cell lines shows multiple hybridizing DNA bands. This suggests that there are multiple laminin receptor genes in the human genome, all of which cross-hybridize. In addition, multiple plaques, each containing unique DNA, were isolated by the plaque hybridization procedure described above from a λ Charon 4A library (obtained from T. Maniatis, Harvard University) of human genomic DNA partially digested with AluI/HaeIII. These findings suggest that the multiple cross-hybridizing laminin receptor genes are not identical. One possibility is that the human genome contains one active laminin receptor gene and several pseudogenes. The mRNAs transcribed from the pseudogenes are sufficiently stable to reach the cytoplasm and can be transcribed in vitro to make cDNA, and their corresponding cDNAs cross-hybridize with authentic laminin receptor cDNA such as λELR1-6 and can be used as alternative hybridization probes in Northern blots. However, due to multiple translational stop codons at the 5' ends of the pseudogenes, laminin receptor protein is not synthesized from them. It is necessary that all isolated laminin receptor cDNAs encode (in open reading frame) the amino acids predicted by λELR4 cDNA sequence to qualify as authentic laminin receptor cDNA.

## Claims

1. A method for diagnosing the aggressiveness of a carcinoma or the effectiveness of an agent for treating cancer cells comprising reacting a sample of a tissue suspected to have said cancer cells with a probe, the diagnosis depending on the degree of hybridisation, characterised in that the probe is obtained from a recombinant cDNA clone encoding cell surface receptor for laminin.

2. A method according to Claim 1, wherein said clone has a sequence corresponding to the cDNA contained in ATCC 67199 encoding laminin cell surface receptor.

3. A method according to Claim 1, wherein said clone has the following sequence: 
CGT GAT GGC ATC TAT ATC ATA AAT CTC AAG AGG ACC TGG GAG AAG
CTT CTG CTG GCA GCT CGT GCA ATT GTT GCC ATT GAA AAC CCT GCT
GAT GTC AGT GTT ATA TCC TCC AGG AAT ACT GGC CAG AGG GCT GTG
CTG AAG TTT GCT GCT GCC ACT GGA GCC ACT CCA ATT GCT GGC CGC
TTC ACT CCT GGA ACC TTC ACT AAC CAG ATC CAG GCA GCC TTC CGG
AAG CCA CGG CTT CTT GTG GTT ACT GAC CCC AGG GCT GAC CAC CAG
CCT CTC ACG GAG GCA TCT TAT GTT AAC CTA CCT ACC ATT GCG CTG
TGT AAC ACA GAT TCT CCT CTG CGC TAT GTG GAC ATT GCC ATC CCA
TGC AAC AAC AAG GGA GCT CAC TCA GTG GGT TTG ATG TGG TGG ATG
CTG GCT CGG GAA GTT CTG CGC ATG CGT GGC ACC ATT TCC CGT GAA
CAC CCA TGG GAG GTC ATG CCT GAT CTG TAC TTC TAC AGA GAT CCT
GAA GAG ATT GAA AAA GAA GAG CAG GCT GCT GCT G[?]G AAG GCA GTG
ACC AAG GAG GAA TTT CAG GGT GAA TGG ACT GCT CCC GCT CCT GAG
TTC ACT GCT ACT CAG CCT GAG GTT GCA GAC TGG TCT GAA GGT GTA
CAG GTG CCC TCT GTG CCT ATT CAG CAA TTC CCT ACT GAA GAC TGG
AGC GCT CAG CCT GCC ACG GAA GAC TGG TCT GCA GCT CCC ACT GCT
CAG GCC ACT GAA TGG GTA GGA GCA ACC ACT GAC TGG TCT TAA GCT
GTT CTT GCA TAG GCT CTT AAG CAG CAT GGA AAA ATG GTT GAT GGA
AAA TAA ACA TCA GTT TCT.
 in whole, or in part.

4. A method according to any preceding Claim, wherein said probe has the ability to hybridise to laminin receptor mRNA.

5. A method according to any preceding Claim, wherein said probe encodes a peptide fragment consisting essentially of a sequence selected from: 
Pro-Thr-Glu-Asp-Trp-Ser-Ala-Gln-Pro-Ala-Thr-Glu-
Asp-Trp-Ser-Ala-Ala-Pro-Thr-Ala;
Met-leu-Ala-Arg-Glu-Val-Leu-Arg; and
Leu-Leu-Leu-Ala-Ala-Arg-Ala-Ile-Val-Ala-Ile
 or a fragment thereof capable of binding to laminin, the probe being sufficient to identify a laminin receptor nucleic acid base sequence by Southern blotting.

6. A method according to any preceding Claim, wherein said probe is capable of identifying laminin receptor mRNA or a laminin receptor gene by Southern blotting.

7. A method according to any preceding Claim for diagnosing the aggressiveness of a carcinoma, wherein purified RNA from said carcinoma is reacted with said probe.

8. A method according to any preceding Claim, wherein the probe is detected chromogenically or by radiolabelling.
